# EUROPEAN PATENT APPLICATION

(11) **EP 3 560 540 A1**
(43) Date of publication of application: **30.10.2019**
(21) Application number: 17888214.8
(22) Date of filing: 11.10.2017
(51) Int. Cl.: A61M 25/00

(54) **DOUBLE-LAYERED DRAINAGE TUBE**

(30) Priority: 26.12.2016 CN 201611218049
(71) Applicant: Innovex Medical Co., Ltd., Shanghai 201210 (CN)
(72) Inventor: ZHENG, Zhongwei, Shanghai 201210 (CN); GONG, Biao, Shanghai 201210 (CN); YUAN, Qing, Shanghai 201210 (CN); WANG, Pan, Shanghai 201210 (CN); YAN, Hang, Shanghai 201210 (CN)
(74) Representative: Wang, Bo
(86) International application number: PCT/CN2017/105627
(87) International publication number: WO 2018/120982

(57) **Abstract**

A double-layered drainage tube (1), composed of an inner layer (11) and an outer layer (12) closely attached to each other, wherein the inner layer (11) is made of a hydrophobic material, and the outer layer (12) is made of a human biocompatible material. It can be ensured that liquids are not linked to an inner wall, thereby preventing bile adhesion from causing drainage tube clogging, and an outer wall thereof is soft and elastic and has a smooth surface, and has a good compatibility with body tissues without causing inflammation. Moreover, in the case where there is no middle layer of a stainless steel wire, the diameter and cost of an outer tube are reduced and the insertion difficulty is reduced, and the inner diameter of the drainage tube (1) is increased, and the effective drainage cross-sectional area of the drainage tube is increased.

## Description

### FIELD OF THE INVENTION

The invention relates to the field of medical device technology, in particular to a double-layered drainage tube.

### BACKGROUND OF THE INVENTION

With the continuous development of endoscopic techniques and the continuous improvement of its accessories, duodenoscopy has become an important measure for the treatment of hepatobiliary and pancreatic diseases, including common bile duct stones, bile duct injury, bile duct stricture, and biliary complications after liver transplantation, etc. The contents of duodenoscopy include: endoscopic retrograde cholangiopancreatography (ERCP) and therapeutic ERCP. Therapeutic ERCP includes endoscopic sphencterotomy (EST), endoscopic biliary drainage (EBD), endoscopic nose biliaty drainage (ENBD), Endoscopic retrograde pancreatodrainage (ERPD) and corresponding endoscopic sputum surgery. With the development of therapeutic ERCP (endoscopic retrograde cholangiopancreatography), endoscopic biliary drainage as the preferred bile duct drainage option has been approved by most clinicians because of its minimal trauma, less complications, and reproducibility compared with conventional techniques.

"Bile duct drainage tube", also known as "biliary tube drainage tube", is mainly used for bile drainage after endoscopic biliary stone surgery and endoscopic gallbladder removal surgery, and is used with duodenal endoscopy, by inserting an indwelling drainage tube as temporary bile drainage. It is a simple and effective method for relieving obstruction. It is mainly used for benign and malignant bile duct obstruction to reduce yellow after operation, diagnosis and treatment of suppurative cholangitis, and other diseases requiring bile duct drainage. A variety of plastic drainage tubes and metal drainage tubes have been used in the prior art for bile duct drainage. Compared to metal drainage tubes, plastic drainage tubes are less expensive, more distorted, and easier to insert. However, both plastic drainage tubes and metal drainage tubes are prone to blockage, have poor biocompatibility, have insufficient strength to insert into the bile duct or easy to cause inflammation and other problems.

The above problems have been studied in the prior art, and a T shaped combined drainage tube for a biliary tract is disclosed in Reference 1 (CN2531802Y), the main body part of the drainage tube is a T shaped tee made of polyvinyl chloride or latex or silica gel material, which is special in that the long arm of the T shaped tee is covered by one layer of film composed of natural red rubber material. The two short arms of the drainage tube are retained in the abdominal chamber during an operation. Polyvinyl chloride or latex or silica gel material is less reactive to biliary tissue, can avoid adverse effects on the biliary tract, and does not cause excessive inflammatory reactions. At the same time, the abdominal sinus is formed early to achieve early extubation by the natural red rubber film in the abdominal chamber, which is on the long arm surface of the drainage tube and strong tissue reactive.

Reference 2(CN1843516A) has been further improved on the basis of reference 1, which only needs to provide a composite layer different from the outer surface layer of the stent body or the stent on the outer surface of one arm of the T-shaped drainage tube holder body. It can realize good effects that the outer surface layer of two arms of the T-shaped drainage tube stent body retained in the body during the operation, is not only compatible with the body and has little damage to the biliary tract stimulation, but also has relatively poor compatibility with the body tissue and can quickly stimulate the formation of sinus around the drainage tube.

However, the above references are all related to the T-type drainage tube, which is mainly used for biliary incision surgery. In order to achieve early extubation, it is as far as possible to promote the formation and stability of the sinus on the basis of ensuring the biocompatibility of the T-type drainage tube and the body. However, the drainage tube used in ERCP surgery does not need to consider the problem of sinus formation, and the biocompatibility of the polyvinyl chloride material used is poor, which can cause damage to the body and cause complications such as inflammation and hyperplasia.

A bonding layer for a surface coating of a medical device is disclosed in Reference 3 (CN1250382A) by coating the medical device surface such as a drainage tube with a thin polymer layer of a suitable composition such that the thin layer bonds well to the substrate surface, and such that succeeding coated layers will be strongly bonded to the thin polymer layer. The device is then coated with other layers designed to enhance performance and for biocompatibility of the medical device. Such layers may include medicated coatings which can serve as surface reservoir for physiologically active agents to release efficacious concentrations of such agents near the surface of the device; hydrogel coating to provide surface lubricity, color containing coatings, abrasion resistant coatings, combinations of one or more of the above, and other coating intended to enhance the performance of the device. It satisfies a long felt need for a thin well bonded lubricious coating for indwelling medical devices. However, by feedback in practice, there is a problem for the biliary drainage tube that it is easy to fall off and it is difficult to meet the needs of long-term use since it needs to be left in the digestive system for a long period of time even if the bonding layer of the above coating layer is used.

Reference 4 (see "Indications and Technical Methods of Endoscopic Biliary Stenting Using the Double layer Stent", Igarashi Y, Miki K, New Challenges in Gastrointestinal Endoscopy, Springer Japan, 2008: 417-422) discloses a double-layered drainage tube used in ERCP surgery. The double-layered drainage tube is actually composed of three layers, and the inner layer is Teflon which is specially processed and chemically smoothened. The inner surface is very smooth, avoiding the adhesion of bile or proteins; the outer layer is a polyamide elastomer, which is used to provide the necessary strength to the drainage tube; there is also a layer of stainless steel mesh between them to ensure that the drainage tube has a certain expansibility. In this way, the inner wall of the drainage tube does not adhere to the bile, and the outer wall has good biocompatibility with the body tissue while maintaining the expansibility of the drainage tube. However, such a three-layer structure, in addition to causing an increase in cost, the middle stainless steel mesh also increases the thickness of the wall of the drainage tube, which actually reduces the effective drainage cross-sectional area inside the drainage tube, or enlarges the overall diameter of the drainage tube, resulting in the problem that the insertion and extubation are difficult and the patient's pain is increased.

Therefore, it is urgent to provide a double-layered drainage tube which has a simpler structure and at the same time realizes that the inner wall does not adhere to the bile, and the outer portion has good biocompatibility with the body tissue.

### SUMMARY OF THE INVENTION

The technical problem to be solved by the present invention is to provide a double-layered drainage tube which has a simple structure and at the same time realizes that the inner wall does not adhere to the bile, and the outer portion and the body tissue have good biocompatibility.

In order to solve the above technical problems, the double-layered drainage tube provided by the present invention is composed of an inner layer and an outer layer closely attached to each other, the inner layer is made of hydrophobic material, the outer layer is made of human biocompatibility material. The inner layer is a hydrophobic material, which can ensure that the inner wall does not hang liquid and avoid the clogging of the drainage tube caused by the adhesion of bile. The outer wall is made of human biocompatible material, which can be left in the human body for a long time, has good compatibility with the body tissue, does not cause inflammation, and ensure the smoothness and safety of the drainage tube.

Further, the human biocompatible material is selected from one or more of polyurethane, polyethylene, silicone rubber or polyamide.

Further, the human biocompatible material is preferably a polyurethane.

Further, the thickness ratio of the outer and inner layers is 10∼1000:1; preferably 50∼ 500:1; optimal selection 100∼200:1.

Further, the hydrophobic material is preferably a fluoropolymer.

Further, the fluoropolymer is preferably a polytetrafluoroethylene or fluorinated ethylene propylene copolymer.

Further, the hardness of human biocompatibility materials is preferably from 85A to 70D, more preferably from 95A to 60D.

Further, the outer layer is further coated with a functional coating, and the functional coating is a hydrophilic coating and/or bioactive coating.

Further, the drainage tube is one of a straight type, a 1/3 bend type, a middle bend type, a horn type or a pig tail type.

Further, the drainage tube is provided with a development mark.

In summary, the double-layered drainage tube according to the present invention is composed of an inner layer and an outer layer closely attached to each other, the inner layer is made of hydrophobic material, the outer layer is made of human biocompatibility material. It can ensure that the inner wall does not hang liquid and avoid the clogging of the drainage tube caused by the adhesion of bile. The outer layer is soft, elastic with smooth surface, has good compatibility with the body tissue, and does not cause inflammation. Compared with the prior art, it does not contain middle layer of stainless steel wires, which reduces the diameter of the outer tube, reduces the difficulty of insertion, increases the inner diameter of the drainage tube, improves the effective drainage cross-sectional area of the drainage tube, has a simple structure and reduces manufacturing cost.

### BRIEF DESCRIPTION OF THE DRAWINGS

The present invention will be further described in detail below with reference to the accompanying drawings and specific embodiments:
Fig. 1 is a schematic structural view of a drainage tube according to an embodiment of the present invention.
Fig.2 is a partially enlarged cross-sectional view showing an embodiment of the present invention.

The component numbers are as follows:
1-Drainage tube
11-Inner Layer
12-outer layer

### DETAILED DESCRIPTION OF THE INVENTION

Preferred embodiments of the present invention will now be described in detail in conjunction with the drawings. Although the description of the present invention will be described in conjunction with the various embodiments, it is not intended that the features of the invention are limited to the embodiments. Rather, the invention is described in connection with the embodiments so as to cover other alternatives or modifications that are possible in the embodiments of the invention. In order to provide a thorough understanding of the present invention, many specific details are included in the following description. The invention may also be practiced without these details. In addition, some specific details are omitted in the description in order to avoid obscuring or obscuring the present invention.

In addition, "upper", "lower", "left", "right", "top", and "bottom" used in the following description are set to better describe the preferred embodiment of the present invention. It should not be construed as limiting the invention.

### Embodiment 1

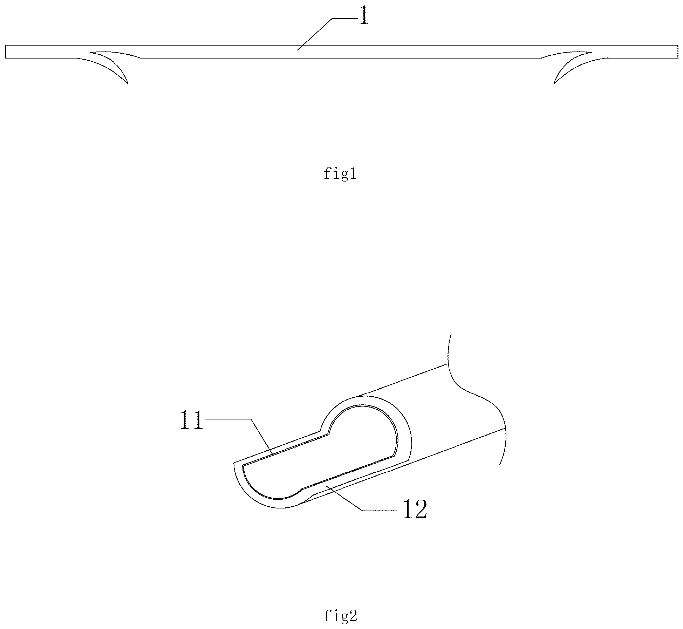

As shown in FIG. 1 and FIG. 2, the double-layered drainage tube provided by the present invention is composed of an inner layer 11 and an outer layer 12 closely attached to each other. The inner layer 11 is made of hydrophobic material, and the outer layer 12 is made of human biocompatibility material. The inner layer 11 is a hydrophobic material, which can ensure that the inner wall does not hang liquid and avoid the clogging of the drainage tube caused by the adhesion of bile. The outer wall 12 is made of human biocompatible material, which can be left in the human body for a long time, has good compatibility with the body tissue, does not cause inflammation, and ensure the smoothness and safety of the drainage tube.

Further, the specific human biocompatible material type is not particularly limited, and the human biocompatible material is preferably selected from one or more of polyurethane, polyethylene, silicone rubber and polyamide considering the biocompatibility and cost of each material.

Further, as the outer layer material, the above human biocompatible materials each have advantages and disadvantages. For example, silica gel has a slightly better biocompatibility, but the insertion strength and radial expansibility are poor. It is not easy to insert into the bile duct when it is used as the outer tube material, and it cannot better maintain the radial expansion of the drainage tube when it is squeezed by the inner wall of the chamber in the working position, so that the effective drainage cross-sectional area of the drainage tube is not guaranteed. The biocompatibility of polyethylene material is comparable to silica gel, strength and expansibility of which is slightly better, but its hardness is higher and bendability is poor, which cannot bend under the body temperature according to the needs of the chamber, may penetrate the inner wall during the insertion process or during the later use, and may cause complications such as inflammation and hyperplasia. The polyamide used in Reference 4 has good biocompatibility, but its water absorption is large, dimensional stability is affected to some extent in the complex internal environment of the human body, and its biocompatibility, strength and radial expansibility are much inferior to polyurethane, and the insertion strength, radial expansion, and developability under X-rays of the drainage tube are reduced in the absence of a mid-layer stainless steel wire.

In the present invention, the polyurethane is preferably used as an outer layer material. The polyurethane has better compatibility with a hydrophobic material such as a fluoropolymer than the other biocompatible materials, and the interface bonding strength is high. The polyurethane material in the face of human temperature has good distortion, which can be bent according to the cavity condition without stabbing the body tissue. The polyurethane is non-toxic and can be left in the human body for a long time, has good compatibility with the body tissue, and does not cause inflammation. Most importantly, it has good strength and radial expansion, and it provides better insertion strength, ensures that the drainage tube does not collapse during use, and ensures smooth flow of the drainage tube even in the absence of the middle layer of stainless steel wire. Moreover, the developing ability of the polyurethane under the X-ray is good, and the drainage tube of the present invention can be clearly observed under X-ray even if the stainless steel wire layer is not contained, which is convenient for the operator to accurately place.

Common hydrophobic materials are fluorine-containing or silane-based polymers, but organosilane materials are poor in chemical resistance. When used as a drainage tube, hydrophobic materials are preferably fluoropolymer hydrophobic materials with more stable chemical properties and lower surface energy. Fluoropolymer hydrophobic materials have high heat resistance, chemical resistance, durability and weather resistance, inactivity especially for many solvents, hydrocarbons, various acids and bases, low surface energy (neither oleophylic nor hydrophilic, ie hydrophobic and oleophobic) and hygroscopic properties. Moreover, the strong C-F bond makes such polymers very stable to redox. The prepared drainage tube can be indwelled to the human body for a long period of time, and the physical and chemical properties are stable. The double-sparing performance ensures that bile or other foreign matter does not adhere inside the drainage tube, and the drainage tube is ensured to be smooth. The fluoropolymer can be selected from the group consisting of Polytetrafluoroethylene (PTFE), fluorinated ethylene propylene copolymer (FEP), perfluoroalkoxy polymer (PFA), copolymer of tetrafluoroethylene and ethylene (ETFE), polyvinylidene fluoride (PVDF) and poly Chlorotrifluoroethylene (PCTFE) and the like, but is not limited to the above materials.

Further, the fluoropolymer is preferably a polytetrafluoroethylene or a fluorinated ethylene propylene copolymer. The polytetrafluoroethylene has the characteristics of being acid and alkali resistant, resistant to various organic solvents, and almost insoluble in all solvents. The propylene copolymer is a soft plastic which has lower tensile strength, abrasion resistance and creep resistance than many engineering plastics and has excellent chemical inertness. Compared with other fluoropolymer hydrophobic materials, the above two materials not only have superior double-stranding performance, but also have a smoother inner surface and less friction, which further reduces the possibility of bile wall hanging.

In the present invention, the relative thicknesses of the inner layer 11 and the outer layer 12 are not particularly limited, and the thicknesses of both may be adjusted according to the use and cost of the art.

Further, for the outer layer material, it is necessary to have a suitable hardness. If the hardness is too low, it will be difficult to provide suitable insertion strength and radial expansion performance for the drainage tube. If the hardness is too high, it is easy to stab the inner wall of the muscle body, which will cause complications such as hyperplasia or inflammation. Therefore, the hardness of the outer layer material is preferably 85A to 70D. Further, in addition to the structure of the material itself, the biocompatibility of the material with the body is also related to whether the hardness of the material matches the body tissue. Therefore, the hardness of the human biocompatible material is more preferably 95A to 60D, and it has better biocompatibility with the body tissue within the above range.

In general, as the hydrophobic material of the inner layer 11, such as a fluoropolymer, mechanical properties such as hardness and strength are high and the price is also high. If the thickness of the inner layer is too large, the manufacturing cost of the drainage tube will increase. In addition, as the thickness of the inner layer increases, the hardness of the inner layer 11 will gradually increase, and if the hardness is too high, the difference in hardness from the outer layer 12 is too large, which will affect the interfacial bonding properties of the inner layer 11 and the biocompatible materials such as polyurethane of the outer layer 12. When the inner layer 11 is thinner, the inner layer 11 has better flexibility and bending property, and has better interfacial bonding properties with materials such as polyurethane of the outer layer 12. Preferably, the thickness ratio of the outer layer 12 to the inner layer 11 is from 10 to 1000:1; more preferably, the thickness ratio of the outer layer 12 to the inner layer 11 is from 50 to 500:1; most preferably, the thickness ratio of the outer layer 12 to the inner layer 11 is from 100 to 200:1.

Further, the outer layer 12 of the draft tube is further coated with a functional coating, and the functional coating is a hydrophilic coating and/or a bioactive coating. The hydrophilic coating can attract water molecules to form a "gelatinous" surface on the surface thereof, reduce the resistance of the drainage tube when inserted, and further improve the biocompatibility of the drainage tube and the body tissue. The hydrophilic coating material is not specifically limited, and the coating is selected from a material having a small frictional resistance. For example, it is preferably made of polyvinyl alcohol, polyvinyl pyrrolidone, polyethylene glycol, polyacrylamide, polyacrylic acid, sodium polyacrylate, Poly (2-hydroxyethyl methacrylate), maleic anhydride copolymer, ethylene vinyl alcohol copolymer, 2-methacryloyloxyethylphosphocholine or its copolymer, (2-hydroxyethyl methacrylate)-styrene block copolymer, various synthetic eptides, collagen, hyaluronate , and one or more of cellulose-based copolymers.

Furthermore, it is also possible to coat the bioactive coating of the pharmaceutical ingredient outside the drainage tube. There is no specific limitation on the type of the bio-coating, and a suitable coating can be selected according to the needs of the patient's condition. For example, antithrombotic agents, such as heparin-quaternary ammonium complexes; or antimicrobial agents such as various silver compounds, quaternary ammonium compounds such as benzalkonium chloride, and phenol derivatives such as thymol; or antibiotics such as gentamicin, nourishment Ofloxacin, and rifamycin.

Further, the shape of the drainage tube is not particularly limited. In addition to the straight type shown in FIG. 1 which is commonly used in ERCP surgery, a 1/3 curved type, a medium curved type, a horn type or a pig ail type may be used. The selection of any of the above shape using the double-layer structure of the present invention can achieve the technical effect of the invention that the inner wall does not adhere to the bile, the outer part has good biocompatibility with the body tissue, the structure is simple, and the cost is low.

Further, in order to facilitate the observation by the operator, the developing portion may be further provided on the drainage tube to facilitate positioning during the process of arranging the drainage tube. The shape of the developing portion is not specifically limited as long as easy to observe, and may be annular, strips, dots, or various forms of patterns such as crosshairs. When the drainage tube enters the patient's body, the development department can clearly show the location of the drainage tube, convenient for the operator to better monitor the drainage tube placement situation.

In summary, the double-layered drainage tube according to the present invention is composed of an inner layer and an outer layer closely attached to each other, the inner layer is made of hydrophobic material, the outer layer is made of human biocompatibility material. the inner wall does not hang liquid and avoid the clogging of the drainage tube caused by the adhesion of bile. The outer layer is soft, elastic with smooth surface, has good compatibility with the body tissue, and does not cause inflammation. And in the case of in the case where there is no middle layer of a stainless steel wire, it reduces the diameter of the outer tube and the cost, decreases the difficulty of insertion, increases the inner diameter of the drainage tube, and improves the effective drainage cross-sectional area of the drainage tube.

The above description of the embodiments of the present invention is intended to illustrate the invention of the present invention, and is not intended to limit the scope of the claims. The above-described embodiments can be easily modified and modified by those skilled in the art in light of the inventive concept of the invention, and the modifications and modifications within the scope of the invention are included in the scope of the appended claims.

## Claims

1. A double-layered drainage tube, wherein it is composed of an inner layer and an outer layer closely attached to each other, the inner layer is made of hydrophobic material, the outer layer is made of human biocompatibility material.

2. The drainage tube as claimed in claim 1, wherein the human biocompatible material is selected from one or more of polyurethane, polyethylene, silicone rubber or polyamide.

3. The drainage tube as claimed in claim 1, wherein the human biocompatible material is a polyurethane.

4. The drainage tube as claimed in claim 1, wherein the thickness ratio of the outer layer and the inner layer is 10∼1000:1.

5. The drainage tube as claimed in claim 1, wherein the hydrophobic material is a fluoropolymer.

6. The drainage tube as claimed in claim 1, wherein the fluoropolymer is a polytetrafluoroethylene or fluorinated ethylene propylene copolymer.

7. The drainage tube as claimed in claim 1, wherein the hardness of the human biocompatible material is from 85A to 70D.

8. The drainage tube as claimed in claim 1, wherein the outer layer is further coated with a functional coating, and the functional coating is a hydrophilic coating and/or bioactive coating.

9. The drainage tube as claimed in claim 1, wherein the drainage tube is one of a straight type, a 1/3 bend type, a middle bend type, a horn type or a pig tail type.

10. The drainage tube as claimed in claim 1, wherein the drainage tube is provided with a development mark.
